# EUROPEAN PATENT APPLICATION

(11) **EP 2 495 002 A1**
(43) Date of publication of application: **05.09.2012**
(21) Application number: 10826255.1
(22) Date of filing: 26.07.2010
(51) Int. Cl.: A61M 5/31, A61M 5/178, B05C 17/01, B65D 83/00

(54) **SYRINGE, DEVICE FOR PRODUCING SYRINGE BODY, AND METHOD FOR PRODUCING SYRINGE BODY**

(30) Priority: 28.10.2009 JP 2009247664
(71) Applicant: Ajinomoto Co., Inc., Tokyo 104-8315 (JP)
(72) Inventor: MURAMATSU, Yasuhiro, Shizuoka-shi Shizuoka 424-0911 (JP)
(74) Representative: Nicholls, Kathryn Margaret
(86) International application number: PCT/JP2010/004737
(87) International publication number: WO 2011/052115

(57) **Abstract**

The sliding property at the inner peripheral surface of a syringe body is improved by means of a simple configuration, and smooth and stable slidability can be ensured for a gasket. A syringe (1) is equipped with a synthetic resin syringe body (3) for injecting a liquid (L), a plunger (5) for pushing out the liquid (L) that was injected into the syringe body (3), and a gasket (9) which is attached to the tip (7) of the plunger (8), wherein the inner peripheral surface (14) of the syringe body (3) is coated with silicone, and is subjected to corona discharge treatment after the syringe body (3) is coated with silicone.

## Description

### TECHNICAL FIELD

The present invention relates to a syringe equipped with a syringe body for injecting a liquid, a plunger for pushing out the liquid injected into the syringe body, and a gasket attached to the tip of the plunger. The present invention also relates to a device for producing the syringe body applied to the syringe, and further relates to a method of producing the syringe body carried out by means of using that device for producing the syringe body.

### BACKGROUND ART

There are syringes with enclosed medical solution, etc. (also referred to as "prefilled syringes"), used for the purpose of injecting liquid such as medical solution into a human body. Typical syringe is composed of a syringe body (also referred to as "syringe barrel") for injecting liquid, a plunger for pushing out the liquid which has been injected into the syringe body, and a gasket attached to the tip of the plunger.
One of the important factors for determining the performance of the syringe is the sliding property of the contact surfaces between the syringe body and the gasket. Poor sliding property of these contact surfaces would increase the slide friction of the gasket during slow movement, which would further cause shuddering friction and deteriorate the smooth movement of the gasket. Thus, when a syringe having poor sliding property of the contact surfaces is used of constant-rate infusion apparatus such as hemodialysis system, any wrong warning signal would occur due to excess of friction limit, or liquid would leak from the excessively deformed gasket.

One of the main reasons for deteriorating the sliding property of these contact surfaces is that most syringe bodies have been formed by using a material such as cyclic polyolefin resin, which is a synthetic resin having poor sliding property. To cope with this problem, according to prior art, there has been a technique of spraying and coating silicone on the inner wall surface of the syringe body for improving the sliding property of the contact surfaces. This prior art is shown in the Patent Document 1 as below.
Further, there are still other techniques suggested for improving the sliding property of the contact surfaces, such as forming dimples on any part of the gasket, or applying laminate coating treatment to the gasket surface. Also, although not having a direct relation to the improvement of sliding property of the contact surfaces, there is another technique of corona discharge treatment on the inner peripheral surface of the syringe body, which basically serves for preventing bubbles generated inside the liquid of the syringe body. This prior art is also shown in the Patent Document 2 as below.

### PRIOR ART DOCUMENTS

### PATENT DOCUMENTS

Patent Document 1: Japanese Unexamined Patent Publication No. Hei 7-149544; and
Patent Document 2: International Publication No. 2008/075460.

### SUMMARY OF THE INVENTION

### PROBLEMS TO BE SOLVED BY INVENTION

However, after coating with silicone on the inner peripheral surface of the syringe body and loading with medical solution, when this syringe body should be preserved, the silicone would become agglutinated and exist in the uneven state, which would deteriorate the sliding property of the contact surfaces. The deterioration of the sliding property of the contact surfaces cannot be remedied by increasing the amount of silicone coated on the inner peripheral surface of the syringe body, or even by increasing the amount of silicone coated on the surface of the gasket. Further, there was an attempt of applying corona discharge treatment to the inner peripheral surface of the syringe body for the purpose of strengthening adhesion of silicone to the inner peripheral surface of the syringe body. However, this attempt resulted in the adverse effect, that the agglutination of silicone emerged more prominently.

As for other techniques such as forming dimples any part of the gasket, or applying laminate coating treatment to the surface of the gasket, these techniques increase production cost of gasket, and require still higher separate technology of prohibiting wrinkles on the laminating films. In particular, when there is any wrinkle on the laminating film, the wrinkled part would become a gap, which would cause leaking of medical solution, etc. from the syringe body.

In the light of the above problems, it is an object of the present invention to solve the problems of the prior arts as discussed above. To achieve the object mentioned above, the present invention provides a user-friendly syringe body of which sliding property of the inner peripheral surface has been improved by simple and low-cost structure, and whereby the slidability of gasket has also been improved. The present invention also provides a device for producing the syringe body applied to that syringe, and further provides a method of producing the syringe body carried out by means of using that device for producing the syringe body.

### MEANS TO SOLVE THE PROBLEM

To achieve the objects mentioned above, according to claim 1 of the present invention, there is a syringe equipped with a synthetic resin syringe body for injecting a liquid, a plunger for pushing out the liquid that was injected into the syringe body, and a gasket which is attached to the tip of the plunger, wherein the inner peripheral surface of the syringe body is coated with silicone, and is subjected to corona discharge treatment after the syringe body is coated with silicone.

According to claim 2 of the present invention, with regard to the syringe of claim 1, the syringe body is formed by cyclic olefinic copolymer.

According to claim 3 of the present invention, there is a device for producing a syringe body, comprising a forming device for forming a syringe body, a silicone coating device for coating silicone on the inner peripheral surface of the formed syringe body, and a corona discharging device for applying corona discharge treatment to the inner peripheral surface of the syringe body coated with silicone.

Further, according to claim 4 of the present invention, there is a method for producing a syringe body, comprising a syringe body forming step for forming a syringe body, a silicone coating step for coating silicone on the inner peripheral surface of the formed syringe body, and a corona discharge treatment step for applying corona discharge treatment to the inner peripheral surface of the syringe body coated with silicone.

The invention as discussed above has the following merits. First, according to the syringe of the present invention, because the inner peripheral surface of the syringe body is coated with silicone, the inner peripheral surface of the syringe body becomes smooth, and when the gasket is accommodated in the syringe body, the sliding property will improve. Further, because of the corona discharge treatment, which is done after coating of silicone and before loading of medical solution, the agglutination of silicone may be prohibited, whereby the even state of silicone may be maintained and the smooth and stable slidability can be ensured for a long time. Thus the syringe of simple and low-cost configuration, with the gasket having a good slidability, may be provided.

When the syringe body is formed by cyclic olefinic copolymer, the transparency of the syringe body may improve, whereby the good visibility from the outside may be secured. Further, although the sliding property of cyclic olefinic copolymer is originally poor, such property would improve, and the good sliding property of the cyclic olefinic copolymer may be secured.
According to the device for producing the syringe body of the present invention, by means of simple addition of corona discharging device to the production line of conventional syringe body, it is possible to produce syringe body effectively and at lower cost, having good slidability when accommodating the gasket.
Further, according to the method for producing the syringe body of the present invention, when carrying out the producing method of conventional syringe body, by means of simple addition of corona discharge treatment step after the silicone coating step, it is also possible to produce syringe body effectively and at lower cost, having good slidability when accommodating the gasket.

### EFFECT OF THE INVENTION

According to the present invention, the syringe, the device for producing the syringe body, and the method for producing the syringe body are capable of improving the sliding property of the inner peripheral surface of the syringe body with simple and low-cost structure, whereby the good slidability of the gasket may be secured. Further, the good slidability of the gasket may also be secured continuously after loading and preserving the medical solution in the syringe body.

### BRIEF DESCRIPTION OF DRAWINGS

[Figure 1] A side view of an assembled syringe according to an embodiment of the present invention.
[Figure 2] A side sectional view of the syringe body according to the embodiment of the present invention.
[Figure 3] A side sectional view showing a forming step of syringe body used in a method for producing the syringe body according to an embodiment of the present invention.
[Figure 4] A side sectional view showing a silicone coating step used in a method for producing the syringe body according to the embodiment of the present invention.
[Figure 5] A side sectional view showing a corona discharging treatment step used in a method for producing the syringe body according to the embodiment of the present invention.
[Figure 6] A half sectional view cut vertically along the center of the syringe body and the gasket, used in a slidability evaluation test for the purpose of examining the effect of the present invention.
[Figure 7] An explanatory table graphically showing the result of slidability evaluation test when the corona discharge treatment was carried out before silicone coating.
[Figure 8] A graphic chart showing the relation between the slide friction and the displacement value when the corona discharge treatment was carried out before silicone coating.
[Figure 9] An explanatory table graphically showing the result of slidability evaluation test when the corona discharge treatment was carried out after silicone coating.
[Figure 10] A graphic chart showing the relation between the slide friction and the displacement value when the corona discharge treatment was carried out after silicone coating.

### BEST MODE FOR CARRYING OUT THE INVENTION

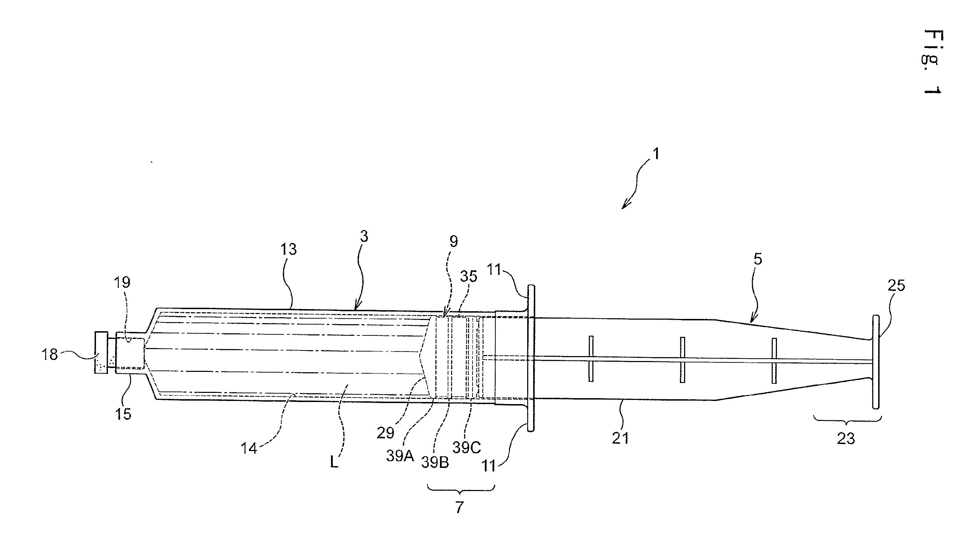

An embodiment of the present invention will now be discussed with reference to Fig. 1 through Fig. 6.
There is a syringe 1 according to the present invention, which is used for example as being attached to a constant-rate infusion apparatus such as hemodialysis system. When the syringe 1 contains, for example, 20 ml of liquid L in a syringe body 3, by attaching this syringe 1 to the constant-rate infusion apparatus, the liquid L of the syringe body 3 will be automatically and constantly injected into a human body for about five hours.

The syringe 1 is equipped with a syringe body 3 for injecting the liquid L, a plunger 5 for pushing out the liquid L that was injected into the syringe body 3, and a gasket 9 which is attached to a tip 7 of the plunger 5.
the syringe body 3 is made of transparent cylindrical-shape container of which one end side being closed, for example rigid plastic material such as cyclic olefinic copolymer, having good transparency and visibility.

The other end side of the syringe body 3 is open-ended, having a flange-shaped finger grip 11 expanding outwardly. The one end surface of the syringe body 3, namely the closed end side, has a nozzle 15 formed at a diameter smaller than that of a barrel 13 of the syringe body 3. As illustrated in Fig. 1, before using, there is a cap 18 attached to the nozzle 15, in order to seal an opening 19 of the nozzle 15.
Likewise the syringe body 3, the plunger 5 is also made of rigid plastic material, etc. The plunger 5 is equipped with a rod 21 inserted into the barrel 13 of the syringe body 3, a depressing plate 25 (e.g. in the shape of disk) formed at a base end portion 23, and an unillustrated male thread portion provided at the tip 7 and serving for attaching of the gasket 9.

The gasket 9 is made of elastic synthetic resin material, etc., such as butyl rubber. The gasket 9 has a closed cap-shaped head surface 29, and the head surface 29 is for example in the shape of umbrella, of which height becomes higher toward the axis center. There is a gasket body 35 around the head surface 29 formed integrally, elongating at a predetermined length toward the base end portion 23 of the plunger 5.
There is a plurality of circular prominent portions 39A, 39B, 39C on the outer peripheral surface of the gasket body 35, respectively in direct and slidable contact with an inner peripheral surface 14 of the syringe body 3.

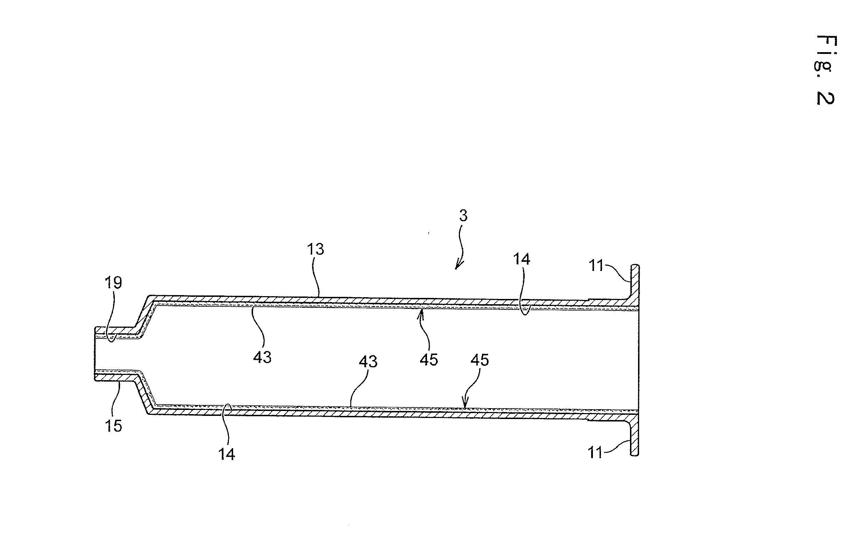

With reference to the syringe 1 of the present invention, one of the most characteristic structures of the present invention is the inner peripheral surface 14 of the syringe body 3 is coated with silicone, and a silicone layer 43 has been formed as illustrated in Fig. 2. After coating of silicone, the surface is further treated by corona discharging, and the top surface of the silicone layer 43 has a surface improved layer 45, on which the agglutination of silicone is prohibited by corona discharge treatment.

Now further explanation of the present invention will be made, as for a device 51 for producing syringe body which produces the syringe body 3 serving as the major structural element of the syringe 1 of the present invention, and as for a method for producing syringe body of the present invention which is carried out by using the device 51 for producing syringe body, with reference to the production flow of the syringe body 3 as below.

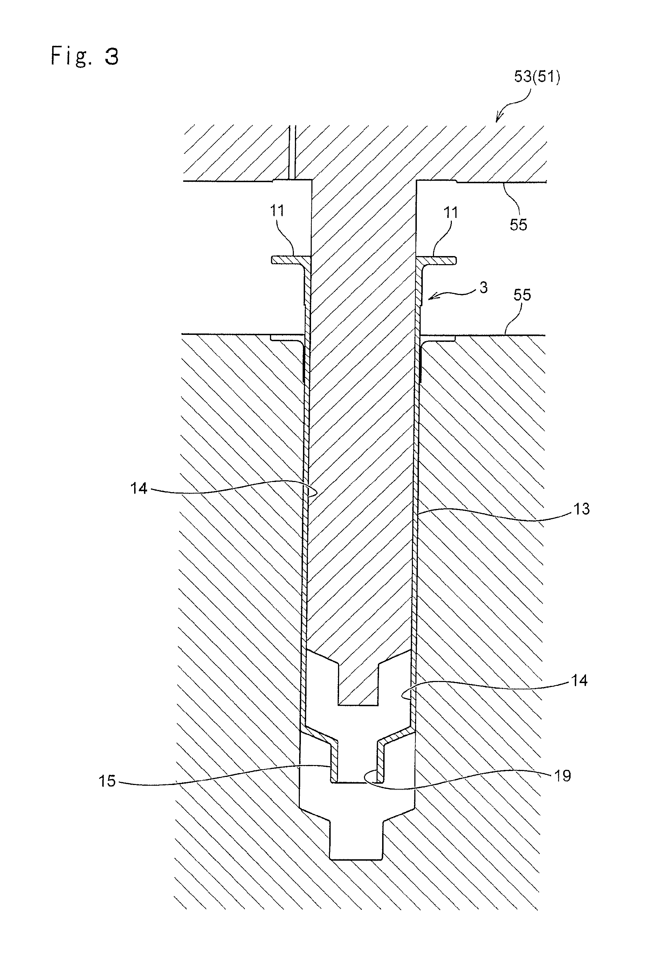

### (1) Forming of Syringe Body (See Fig. 3)

A forming device 53, such as an injection molding machine, is used for forming of the syringe body 3. For example, any melt resin pellet material is poured into a cavity of a mold 55, and after solidification, the syringe body 3 can be obtained.
The forming device 53 is a structural element of the device 51 for producing syringe body of the present invention, and the step of forming the syringe body 3 by using the forming device 53 serves as a syringe body forming step, which is a structural element of the method for producing syringe body according to the present invention.

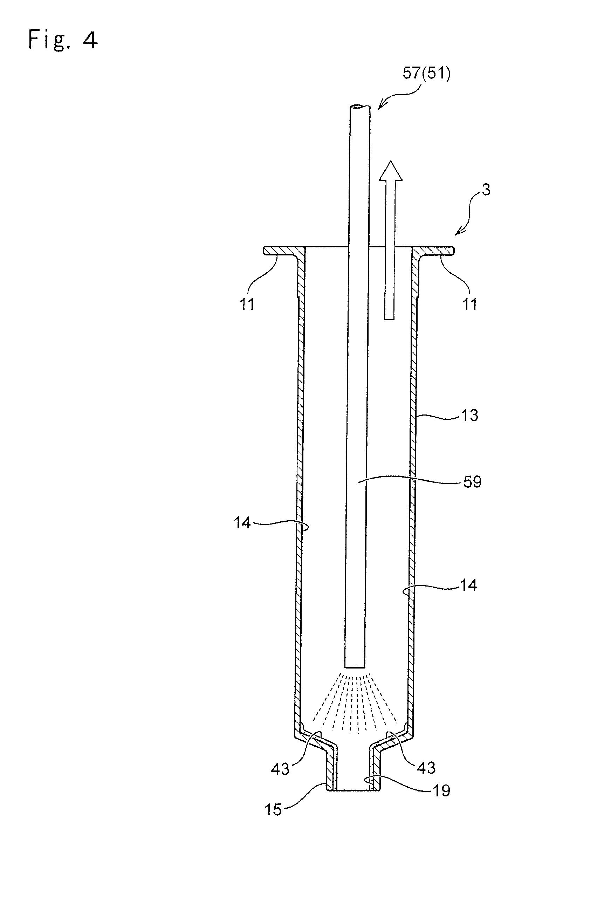

### (2) Coating of Silicone on the Inner Peripheral Surface of the Syringe Body (See Fig. 4)

The syringe body 3 formed by the forming device 53 is then supplied to a silicone coating step, which is also a structural element of the method for producing syringe body according to the present invention. At this step, by using a silicone coating device 57 serving as a structural element of the device 51 for producing syringe body of the present invention, a coating nozzle 59 is inserted into the barrel 13 of the syringe body 3, and the coating nozzle 59 sprays silicone toward the inner peripheral surface 14 of the syringe body 3, whereby the silicone layer 43 is formed.

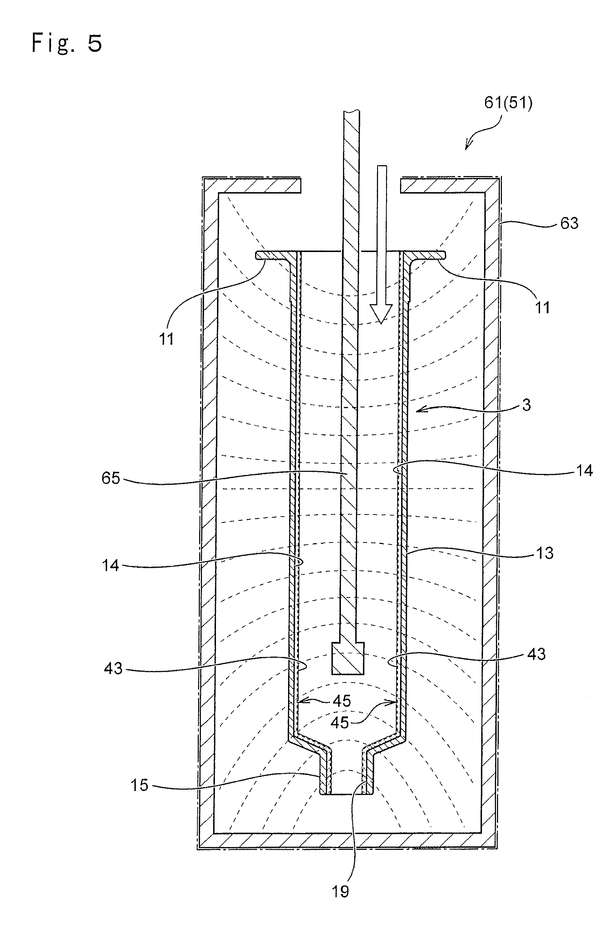

### (3) Corona Discharge Treatment on the Silicone Surface (See Fig. 5)

The syringe body 3, having the silicone layer 43 formed on the inner peripheral surface 14 by the silicone coating device 57, is then supplied to a corona discharge treatment step, which is also a structural element of the method for producing syringe body according to the present invention. At this step, by using a corona discharging device 61 serving as a structural element of the device 51 for producing syringe body of the present invention, the corona discharge treatment is applied to the surface of the silicone layer 43.
As a practical example, the syringe body 3 of which inner peripheral surface 14 has been coated with silicone at the silicone coating step, is placed in a treatment container 63 in a position that the side of the nozzle 15 is inserted first. The treatment container 63 is covered with dielectric material. Further, a stick-shape discharge electrode 65 is inserted into the barrel 13 of the syringe body 3, from the side of open-ended base end portion 23 of the syringe body 3.

Thereafter, an unillustrated high-frequency power supply supplies high-frequency and high-voltage output, and this output is applied to the space between the discharge electrode 65 and the treatment container 63, whereby the corona discharge is generated. Thus, under corona discharge in the treatment container 63, the corona discharge treatment is carried out on the surface of the silicone layer 43 on the syringe body 3.
The surface of the silicone layer 43 after corona treatment is formed as the surface improved layer 45, having remarkable improvement in leakage prohibition performance because of synergetic effect between the physical surface improvement by discharge itself and the chemical surface improvement by generation of polar functional group.

### (4) Cap Fixing; Medical Solution Loading; and Gasket Sealing

After application of corona discharge treatment at the corona discharge treatment step, the cap 18 is fixed to the nozzle 15 of the syringe body 3, whereby the opening 19 is sealed. The syringe body 3 is loaded with a predetermined amount of the medical solution L in the barrel 13. After loading of the medical solution L, the gasket 9 is inserted, in a state that the head surface 29 is inserted first, from the open-ended base end portion of the syringe body 3, so that the gasket 9 may seal the syringe body 3. Thus the open-ended base end portion of the syringe body 3 is sealed, and the syringe body 3 as a final product, loaded with the medical solution L, is obtained.
The device 51 for producing syringe body according to the present invention basically comprises the forming device 53, the silicone coating device 57 and the corona discharging device 61. On the other hand, the method for producing syringe body according to the present invention basically comprises the syringe body forming step, the silicone coating step and the corona discharge treatment step.

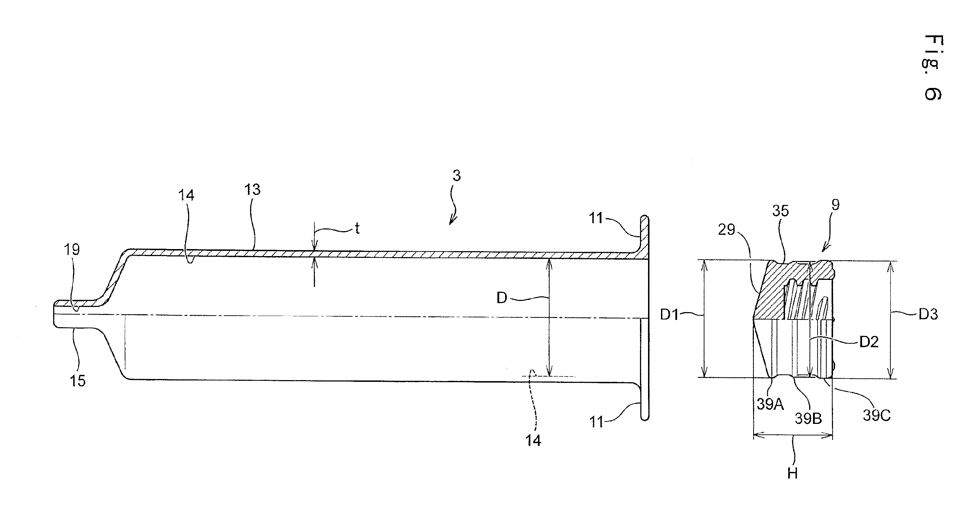

Now the content and result of slidability evaluation test will be explained, which was carried out for the purpose of ascertaining the effect of the present invention as below:
First, according to the present test, the syringe body 3 and the gasket 9 as illustrated in Fig. 6 were used. As for the syringe body 3, the inner diameter D was 20.1 mm, barrel thickness t was 1 mm, the material was cyclic olefinic copolymer (COC), and the liquid content was 20 ml. As for the gasket 9, the first peak diameter D1 was 20.55 mm, the second peak diameter D2 was 20.35 mm, the third peak diameter D3 was 20.60 mm, the height H was 14 mm, and the material was butyl rubber. By using them, the tests were carried out regarding: (A) Comparison test between the case of "with" or "without" the corona discharge treatment before the silicone coating; and (B) Comparison test between the case of "with" or "without" the corona discharge treatment after the silicone coating.

Those tests were carried out by using a compression tester. Each syringe body 3, serving as a sample, contained 20 ml of the medical solution L and was sealed by the gasket 9, and kept for one month at room temperature. The slidability evaluation test was carried out under the condition that the injection speed was 0.5 mm/min (1 ml/h) and the pressure inside the syringe was 338 mmHg.

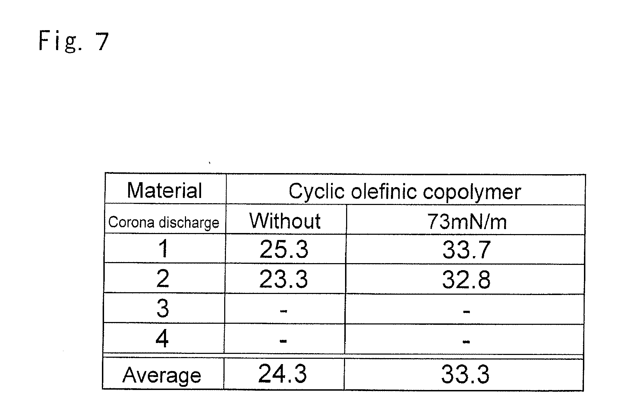

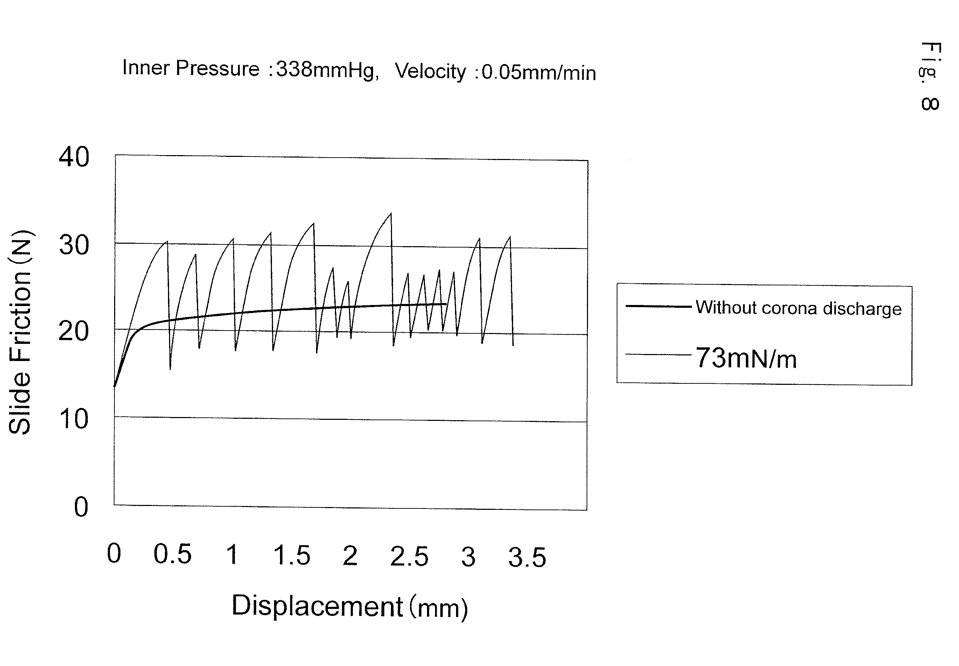

### (A) Corona Discharge Treatment Before Silicone Coating (See Fig. 7, Fig. 8)

According to this comparative test, the treatment level of corona discharge was set as Wetting Index 73 mN/m, and the slide friction (N) was measured by carrying out the test "with" the corona discharge treatment, and also the test "without" the corona treatment, each test twice, whereby the respective average values were obtained. The Wetting Index is an index value for measuring wettability, used in Wetting Tension Test Method (JIS K6768), and shows the surface improvement level by means of corona discharging.
Figs. 7 and 8 show the result of the above test. The slide friction (N) became larger in the case of "with" the corona discharge treatment. This may be understood as the adverse effect, and as illustrated in Fig. 8, the silicone agglutinated more significantly.

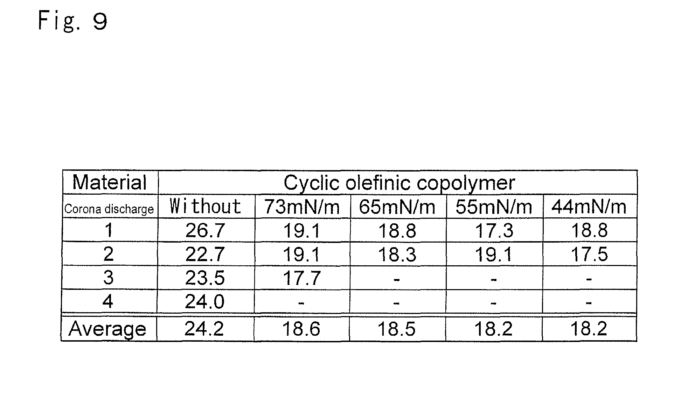

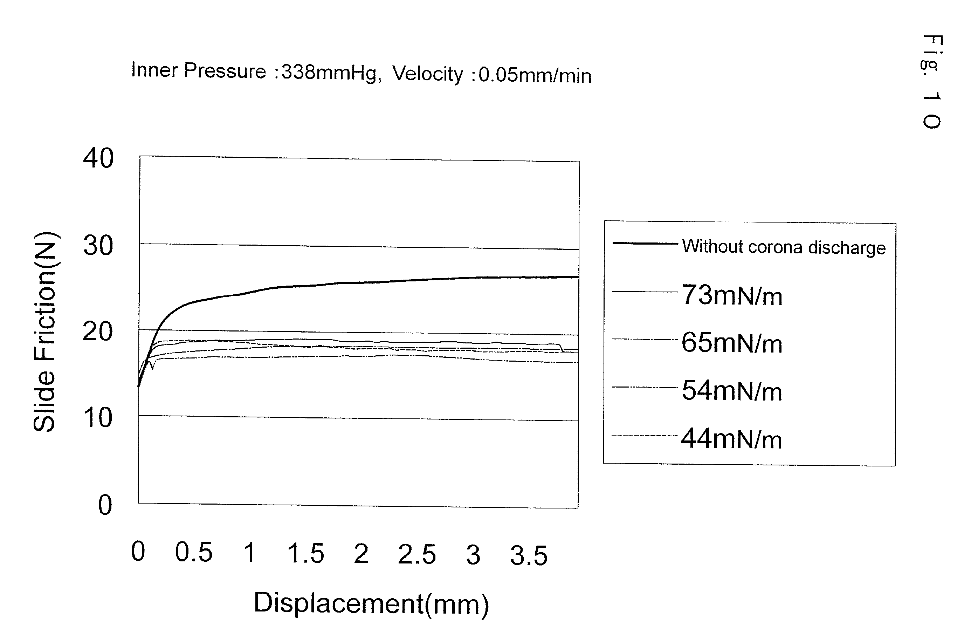

### (B) Corona Discharge Treatment After Silicone Costing (See Figs. 9, 10)

According to this comparative test, four treatment levels of "with" corona discharge were set as Wetting Index 73 mN/m, 65 mN/m, 55 mN/m and 44mN/m, and regarding each of the levels, as well as for the case of "without" the corona discharge treatment, the slide friction (N) was measured by carrying out the test for two - four times depending on the level, whereby the respective average values were obtained.
Figs. 9 and 10 show the result of this test. As compared with the case of "without" the corona discharge treatment, the case of "with" the corona discharge treatment had the lower slide friction (N) regardless of the level of the corona discharge treatment. Further, as illustrated in Fig. 10, although there was no significant agglutination of silicone both in the cases of "with" and "without" the corona discharge treatment, it was still found that the case of "with" the corona discharge treatment had the remarkable excellent homogeneity of silicone.

As is clear from the result of the above two comparative tests, the syringe 1 of the present invention, to which the corona discharge treatment has been applied after the silicone coating, because of the corona discharge treatment after the silicone coating, improves the surface of the silicone layer 43, prohibits the agglutination of silicone, and improves the homogeneity of silicone. Accordingly, it was proven from these comparative tests, that the sliding property of the inner peripheral surface 14 of the syringe body 3, obtained by coating of silicone, was maintained and exhibited.

The syringe 1, the device 51 for producing syringe body, and the method for producing syringe body according to the present invention, are not limited to the embodiments as discussed above, and any modification may be made within the scope of the present invention. For example, the synthetic resin material forming the syringe body 3 is not limited to the cyclic olefinic copolymer, and any other rigid plastic material such as cyclic polyolefin (COP) resin or polypropylene (PP) resin may be used. When the syringe body is formed by any of these other materials, the above effect may be obtained by applying the same treatment as discussed above.
Further, the fixing of the cap 18, which is to be carried out after the corona discharge treatment, may also be done with regard to the syringe body 3 immediately after forming, that is, before the silicone coating. The fixing may also be done with regard to the syringe body 3 after the silicone coating and before the corona discharge treatment.

### INDUSTRIAL APPLICABILITY

The present invention may be used in the field of producing and/or using the syringe equipped with the syringe body for injecting the liquid, the plunger for pushing out the liquid injected into the syringe body, and the gasket attached to the tip of the plunger. In particular, the present invention may be applied to the case that the sliding property of the inner peripheral surface of the syringe body should be improved, so that the stable and smooth sliding property of the gasket should be obtained.

### EXPLANATION OF REFERENCE SIGNS

- 1: Syringe

- 3: Syringe body
- 5: Plunger
- 7: Tip
- 9: Gasket
- 11: Finger grip
- 13: Barrel
- 14: Inner peripheral surface
- 15: Nozzle
- 18: Cap
- 19: Opening
- 21: Rod
- 23: Base end portion
- 25: Depressing plate
- 29: Head surface
- 35: Gasket body
- 39: Circular prominent portions
- 43: Silicone layer
- 45: Surface improved layer
- 51: Device for producing syringe body
- 53: Forming device
- 55: Mold
- 57: Silicone coating device
- 59: Coating nozzle
- 61: Corona discharging device
- 63: Treatment container
- 65: Discharge electrode
- L: Liquid
- D: Diameter
- t: Thickness
- D1: First peak diameter
- D2: Second peak diameter
- D3: Third peak diameter
- H: Height

## Claims

1. A syringe equipped with a synthetic resin syringe body for injecting a liquid, a plunger for pushing out said liquid that was injected into said syringe body, and a gasket which is attached to the tip of said plunger, **characterized in that**:
the inner peripheral surface of the syringe body is coated with silicone, and is subjected to corona discharge treatment after said syringe body is coated with silicone.

2. The syringe as claimed in claim 1, **characterized in that** the syringe body is formed by cyclic olefinic copolymer.

3. A device for producing a syringe body, comprising:
a forming device for forming a syringe body;
a silicone coating device for coating silicone on the inner peripheral surface of said formed syringe body: and
a corona discharging device for applying corona discharge treatment to the inner peripheral surface of said syringe body coated with silicone.

4. A method for producing a syringe body, comprising:
a syringe body forming step for forming a syringe body;
a silicone coating step for coating silicone on the inner peripheral surface of said formed syringe body: and
a corona discharge treatment step for applying corona discharge treatment to the inner peripheral surface of said syringe body coated with silicone.
